Europäisches Patentamt

**European Patent Office**

Office européen des brevets

(19)

(11) Veröffentlichungsnummer : **0 001 256**
**B1**

(12) **EUROPÄISCHE PATENTSCHRIFT**

(45) Veröffentlichungstag der Patentschrift :
**25.03.81**

(21) Anmeldenummer : **78100879.2**

(22) Anmeldetag : **13.09.78**

(51) Int. Cl.³ : **A 61 F 13/00// D04B21/20**

(54) **Dauerelastische Netzbinde.**

(30) Priorität : **16.09.77 DE 2741826**

(43) Veröffentlichungstag der Anmeldung :
**04.04.79 (Patentblatt 79/07)**

(45) Bekanntmachung des Hinweises auf die Patenterteilung : **25.03.81 Patentblatt 81/12**

(84) Benannte Vertragsstaaten :
**BE CH FR GB LU NL SE**

(56) Entgegenhaltungen :
**DE - A - 2 056 271**
**US - A - 3 183 685**
**US - A - 3 251 201**
**US - A - 3 570 482**

(73) Patentinhaber : **LOHMANN GmbH & CO KG**
**Irlicher Strasse 55**
**D-5450 Neuwied 12 (DE)**

(72) Erfinder : **Westip, Wilhelm**
**Neuenbaumer Weg 90**
**D-5600 Wuppertal 1 (DE)**

(74) Vertreter : **Splanemann, Rainer et al**
**Patentanwälte R. Splanemann Dr. B. Reitzner Tal 13**
**D-8000 München 2 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

## Dauerelastische Netzbinde

Die Erfindung bezieht sich auf eine dauerelastische Netzbinde, bestehend aus einem Gewirke mit elastischen Fäden als Kette und quer zu dieser verlaufenden unelastischen Fäden als Schuß. Derartige Binden sind durch die CH-A-192927 bekannt.

Bei anderen elastischen Binden mit Baumwollkette bzw. Idealbinden (DIN-Blatt 61632) wird die Dehnbarkeit durch überdrehte Zwirne in der Kettrichtung erreicht, die in dem System 2Z, 2S gelegt sind. Ihre Dehnung ist durch die Schußfadenzahl einstellbar. Als Nachteil dieser Binde wird das begrenzte Rückstellvermögen empfunden. Das heißt, die überdrehten Zwirne (Kreppzwirne) werden so gedehnt, daß die Binde nach mehrmaligem Gebrauch ihre Elastizität verliert. Sie kann durch Waschen nur teilweise regeneriert werden.

Weiterhin ist es bekannt, anstelle der überdrehten Kreppzwirne in der Kettrichtung Kräuselfäden aus Polyamid vorzusehen. Die Dehnbarkeit dieser Binde läßt sich auf ca. 60 bis 200 % einstellen. Die Binde hat aufgrund der dauerhaften Kräuselung der Polyamidfäden ein gutes Rückstellvermögen. Durch Waschen läßt sich eine völlige Regeneration dieser Binde erreichen. Ein Nachteil ist, daß diese Binde einen erheblichen Wärmestau beim Tragen entwickelt.

Bei einer weiteren Gruppe von bekannten elastischen Binden werden die Kettfäden aus Gummi oder Polyurethan oder einer Kombination dieser Fadentypen gebildet. Diese Binden haben ein sehr gutes Rückstellvermögen und lassen sich gut waschen. Die Fadendichte bei derartigen Binden muß so gewählt werden, daß eine Fadenverschiebung bei der Benutzung vermieden wird. Die Binde wird dadurch verhältnismäßig schwer und dick. Hierdurch wird der Tragekomfort beeinträchtigt.

Andere, durch die DE-A-2120607 bekannte, elastische Binden sind durch Wirken hergestellt und enthalten in ihrer Längsrichtung synthetische, gekräuselte Fäden oder Fasern, die eine künstliche Elastizität aufweisen. Außerdem sind in der Längsrichtung noch nicht-dehnbare Fäden oder Fasern, insbesondere Baumwollfäden, verarbeitet. Diese Fäden bilden im wechselnden Rhythmus parallel laufende Ketten, wobei die elastischen Fäden beim Anzetteln auf das Höchstmaß gespannt werden, während die Baumwollfäden oder andere nicht dehnbare Fäden ohne Spannung oder nur in ihrer normalen Streckung verarbeitet werden, so daß nach der Herstellung und dem elastischen Zusammenziehen der synthetischen Fäden ein Schlaffwerden der Maschen eintritt, wodurch die nichtdehnbaren Fäden Wellen bilden. In der Querrichtung zu diesen Fäden werden nicht-dehnbare Fäden vermascht oder verbunden, wodurch eine Maschenbinde entsteht. Die Binde erhält ihre Formelastizität dadurch, daß an den Randkanten vorzugsweise dehnbare synthetische Fäden eingesetzt werden. Diese Maschenbinde hat eine spürbar verbesserte Absorptionsfähigkeit. Sie ist sehr dicht und warm und besitzt aufgrund der Stauchung der unelastischen Längsfäden ein dickes bzw. voluminöses Aussehen. Diese Wirkbinden haben den Nachteil, daß sie in der Breite bei der Längsdehnung stark schrumpfen und zu einem Wärmestau führen können.

Bei der dauerelastischen Netzbinde der eingangs erläuterten Art sind zwischen den gummielastischen Kettfäden unelastische Schußfäden derart angeordnet, daß die Schußfäden zwischen einer ersten und einer zweiten Kette an einer anderen Stelle der zweiten Kette angreifen, als die Schußfäden zwischen der zweiten und einer dritten Kette. Bei einer Dehnung ergibt sich dabei eine wabenförmige Verformung, die bei nur einseitiger Zugbelastung zu einer Schrumpfung des Gewirkes quer zur Zugrichtung führt. Diese Breitenschrumpfung beeinträchtigt die gleichmäßige Auflage der Binde, was ebenfalls als Nachteil empfunden wird.

Es ist deshalb Aufgabe der vorliegenden Erfindung, eine Netzbinde zu schaffen, die besonders leicht und großmaschig ist, bei der aber gleichzeitig die Gefahr der Breitenschrumpfung ausgeschaltet ist.

Zur Lösung dieser Aufgabe wird bei der eingangs näher erläuterten dauerelastischen Binde erfindungsgemäß vorgeschlagen, daß als Ketten voneinander unabhängige und unter Abstand voneinander angeordnete Fransenketten aus unelastischen Fäden vorgesehen sind, die mit einem elastischen Kunststoffaden unterlegt sind, der parallel zu der Fransenkette verläuft, und die unelastischen Schußfäden rechtwinklig zu diesen verlaufen und stets an der gleichen Stelle der Fransenkette aufliegen, an der auch der benachbarte Schuß angreift, und zwischen den Sprüngen von Fransenkette zu Fransenkette mehrmals auf dieser bogenförmig abgelegt sind.

Die US-A-3 570 482 zeigt zwar in Kettrichtung verlaufende Fransen, die jedoch in dem Gewirk dicht nebeneinander liegen und von denen jede Masche mit den beiden benachbarten Maschen der beiden benachbarten Fransen durch einen Schußfaden verbunden ist. Um eine zweidimensionale Dehnbarkeit des Gewirks zu erreichen, ist ein elastischer Faden hin- und hergehend in jede einzelne Franse eingearbeitet und läuft im Bereich der einzelnen Masche im wesentlichen parallel zu dem unelastischen Schußfaden. Das Gewirk kann wasserlösliche Schußfäden enthalten, um so leichter Einzelverbände abtrennen zu können. Für eine Kompressionsbinde ohne Breitenschrumpfung ist das Gewirk gemäß US-A-3 570 482 ungeeignet.

Die DE-A-2 056 271 offenbart einen Schlauchverband zur Fixierung von Wundabdeckungen, die US-A-3 251 201 betrifft ein Salamiwurstnetz und die US-A-3 183 685 beschreibt die Fertigung einer Chantillyspitze, die den an eine elastische Netzbinde von hoher Längsstabilität und ohne Breitenschrumpfung gestellten Anforderungen nicht genügen und damit für diese ungeeignet sind.

Eine derartige Netzbinde eignet sich für die allgemeine Anwendung als Stütz- oder Kompressionsverband bzw. zum Einsatz bei Krampfadern und offenen Beinen in Verbindung mit einer Kompresse. Aufgrund der periodisch durchgehenden Schußlinien hat die Binde eine erhebliche Seiten- und Längsstabilität. Die Binde hat einen kräftigen Stand, obgleich sie leicht und grobmaschig ist. Dennoch ist wegen der stets an derselben Stelle der Fransenkette angreifenden Schußfäden eine Breitenschrumpfung ausgeschaltet.

Die Fransenketten der Längs- bzw. Maschinenlaufrichtung bestehen aus Polyamidfäden und werden mit elastischen Fäden aus Gummi oder Polyurethan allein oder im Gemisch mit anderen einsetzbaren Kunststoffäden hinterlegt, und zwar so, daß die Fransenketten und die elastischen Fäden parallel zueinander in bestimmten Abschnitten aufgelegt werden. Zur Maschenbildung werden diese Fransenketten und elastischen Fäden durch den Schuß, der senkrecht zur Maschinenrichtung verläuft, verbunden. Der Schußfaden wechselt entweder zu einer Nachbarfranse oder aber er überspringt ein oder mehrere Fransenketten, geht dann beim nächsten Schuß wieder zurück und legt mehrere Male auf dem gleichen elastischen Kettfaden bogenförmig auf.

Durch den Schuß, der von Fransenketten zu Fransenketten geht, wird die Binde zu einem Gewirke verbunden. Die kurze Legung des Schußfadens auf dem gleichen Kettfaden erzeugt das netzartige großmaschige Aussehen der Binde. Außerdem wird hierdurch ein breitenstabiles Gewirke erreicht, das im gedehnten Zustand keinen Breitenschrumpf aufweist. Je nachdem, ob nun der Sprung des Schußfadens von einem Kettfaden zum benachbarten oder weiter geht, entsteht ein unterschiedliches Maschenbild, ohne daß jedoch hierdurch die Wirkungsweise der Binde beeinflußt wird.

Da die eingewirkten Schußfäden, wie beispielsweise Baumwolle, gewöhnlich saugfähig sind, kann die Netzbinde genügend Feuchtigkeit aufnehmen und vermeidet so gleichzeitig einen Nässestau.

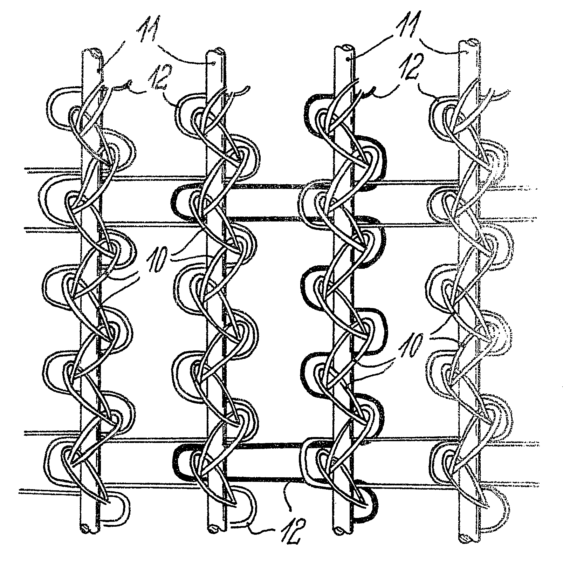

In der Zeichnung ist das Maschenbild eines Ausführungsbeispiels der erfindungsgemäßen dauerelastischen Netzbinde wiedergegeben.

Die fransenbildenden Fäden 10 sind aus Polyamid. Bei diesem Ausführungsbeispiel sind die Fäden 10 mit gestreckt liegenden Polyurethanfäden 11 hinterlegt. Zwischen diesen parallel zueinander und in Maschinenlaufrichtung verlaufenden Fransenketten 10 und Polyurethanfäden 11 werden unelastische Fäden 12, wie beispielsweise Baumwollfäden, so eingeschossen, daß die Fäden 12 gleichmäßig zu Nachbar- oder weiter entfernten Fransenketten verlaufen. Einer der Fäden 12 ist ausgezogen dargestellt, um den Fadenverlauf zu verdeutlichen. Hierdurch entsteht ein symmetrisches Maschenbild. Dieses bewirkt, daß ein Breitenschrumpf vermieden wird, weil die Zugkraft der Schußfäden über die gesamte Fläche verteilt, jeweils am gleichen Punkt einsetzt und rechtwinklig zu den Fransenketten 10 verläuft.

Die Maschengröße der Netzbinde ist abhängig von der Anzahl der Legungen auf einer Fransenkette 10 und von dem Abstand der Fransenketten 10 untereinander.

Der unelastische Schuß kann auch in anderer Weise angeordnet werden. So kann der Schuß zur Nachbarfranse wechseln, dort einige Legungen auf derselben Franse machen und dann erst wieder zurückgehen, um auch hier einige kurze bogenförmige Legungen zu machen. Der Schuß arbeitet dabei immer nur einen Weg und nicht hin und zurück. Der Schuß kann aber auch in dieser Weise zur Nachbarfranse arbeiten und wieder zurück, legt sich dann aber nicht einige Male bogenförmig, sondern arbeitet wie der elastische Polyurethanfaden und wird somit von der Franse umschlungen.

**Ansprüche**

1. Dauerelastische Netzbinde, bestehend aus einem Gewirke mit elastischen Fäden als Kette und quer zu dieser verlaufenden unelastischen Fäden als Schuß, dadurch gekennzeichnet, daß als Ketten voneinander unabhängige und unter Abstand voneinander angeordnete Fransenketten aus unelastischen Fäden (10) vorgesehen sind, die mit einem elastischen Kunststsoffaden (11) unterlegt sind, der parallel zu der Fransenkette (10) verläuft, und die unelastischen Schußfäden (12) rechtwinklig zu diesen verlaufen und stets an der gleichen Stelle der Fransenkette aufliegen, an der auch der benachbarte Schuß angreift, und zwischen den Sprüngen von Fransenkette zu Fransenkette mehrmals auf dieser bogenförmig abgelegt sind.

2. Dauerelastische Netzbinde nach Anspruch 1, dadurch gekennzeichnet, daß die Schußfäden (12) von einer Fransenkette (10) auf die Nachbarkette springen.

3. Dauerelastische Netzbinde nach Anspruch 1, dadurch gekennzeichnet, daß die Schußfäden (12) auf eine entferntere Fransenkette (10) springen.

**Claims**

1. Permanently elastic network bandage, consisting of a fabric with elastic threads as warp and nonelastic threads extending transversely thereto as weft, characterised in that as warps there are provided fringe warps of nonelastic threads (10) independent of one another and arranged spaced apart, which are underlaid with an elastic plastics thread (11) which extends parallel to the fringe warp (10), and the nonelastic weft threads (12) extend at right angles to these and are always applied against the same point of the fringe warp, at which the adjacent weft also engages, and between the jumps from fringe warp to fringe warp are repeatedly laid on this latter in curved form.

2. Permanently elastic network bandage according to Claim 1, characterised in that the weft threads (12) jump from one fringe warp (10) to the adjacent warp.

3. Permanently elastic network bandage according to Claim 1, characterised in that the weft threads (12) jump to a more remote fringe warp (10).

**Revendications**

1. Bande en filet douée d'élasticité permanente formée d'un tissu maillé comportant comme chaînes des fils élastiques et comme trame des fils non élastiques dirigés transversalement à ceux-ci, caractérisée en ce que les chaînes sont des chaînes de frange indépendantes les unes des autres, formées de fils non élastiques (10), espacés les uns des autres et doublés d'un fil élastique de matière synthétique (11) dirigé parallèlement à la chaîne de frange (10) et en ce que les fils de trame non élastiques (12) sont dirigés perpendiculairement aux précédents et s'appliquent toujours au même point de la chaîne de frange que la trame voisine et sont pliés plusieurs fois en arc sur la chaîne de frange, entre les sauts d'une chaîne de frange à l'autre.

2. Bande en filet douée d'élasticité permanente selon la revendication 1, caractérisée en ce que les fils de trame (12) sautent d'une chaîne de frange (10) à la chaîne voisine.

3. Bande en filet douée d'élasticité permanente selon la revendication 1, caractérisée en ce que les fils de trame (12) sautent sur une chaîne de frange (10) plus éloignée.